# EUROPEAN PATENT SPECIFICATION

(11) **EP 0 658 207 B1**
(45) Date of publication and mention of the grant of the patent: **25.09.2002**
(21) Application number: 93917945.3
(22) Date of filing: 29.07.1993
(51) Int. Cl.: C12N 15/82, C12N 15/60, A61H 5/00, C12N 15/56, C12N 15/55, C12N 15/29, C12N 15/31, C12N 15/11, C12N 15/52

(54) **METHOD FOR THE GENETIC CONTAINMENT OF PLANTS**
VERFAHREN ZUR GENETISCHE EINKREISUNG VON PFLANZEN
PROCEDE GENETIQUES POUR L'ENCERCLEMENT DES PLANTES

(30) Priority: 29.07.1992 GB 9216151
(43) Date of publication of application: 21.06.1995
(73) Proprietor: Syngenta Limited, Guildford Surrey GU2 7YH (GB)
(72) Inventor: BRIGHT, Simon, William, Jonathan, Marlow Bucks SL7 2AY (GB); GREENLAND, Andrew, James, Kingswood Court Braywick Road Maidenhead (GB); JEPSON, Ian 41 Grays Road, Berkshire SL1 3QG (GB); PAINE, Jacqueline, Ann, Mary, Crown Wood Bracknell Berkshire RG12 3TQ (GB)
(74) Representative: Huskisson, Frank Mackie
(86) International application number: GB9301605
(87) International publication number: WO94003619

(56) References cited:
- EP-A- 0 332 104
- EP-A- 0 412 006
- EP-A- 0 494 724
- WO-A-90/08828
- WO-A-90/08830
- WO-A-91/03561
- WO-A-91/09957
- WO-A-92/01799
- THE PLANT CELL vol. 3, no. 6 , June 1991 pages 573 - 582 KARLIN-NEUMANN, G.A., ET AL. 'Phytochrome control of the tms2 gene in transgenic Arabidopsis: A strategy for selecting mutants in the signal transduction pathway'

## Description

This invention relates to a method for the containment of plant germplasm. More generally, the invention relates to the molecular control of plant development through to maturity and seed production.

Agriculture uses many crop plants for the production of food for human consumption, for commercial processes yielding products for human consumption, for animal feedstuff production, for the development of industrial products and other purposes. The process involves the planting by the farmer of seed which usually has been purchased from a seed producer. The product produced by the crop be it the whole plant, the seed or fruit of the plant, is harvested and is then used for the various food applications mentioned above.

The supplied hybrid or inbred seed may incorporate novel genetic information introduced by transformation of the crop giving novel agronomic features such as tolerance to herbicides, insect pests, and fungal diseases, improved yield and/or quality of the harvested product, and novel mechanisms for the control of plant fertility. Such improvements made possible through biotechnological research, improve the quality of the plant breeding and improve the agronomic performance of the seed supplied to the farmer.

A problem addressed by the present invention is the containment of crop plants within the area of cultivation. Seeds of cultivated crop plants may be conveyed outside the defined growing area by a number of routes (by birds or small mammals or simply by being dropped during post-harvest transport of a seed crop) where they assume the status of weeds, or they may remain as volunteers in a subsequent crop in later years. It would clearly be appropriate, if it were possible, that cultivated crops be confined to the growing area and prevented from persisting in the wild.

A second agricultural problem addressed by the present invention is that of pre-harvest sprouting. This is a particular problem with small grained cereals where rainfall or high humidity prior to harvest causes seed to begin to germinate whilst still in the ear. It would clearly be advantageous to the farmer, if it were possible, that pre-harvest sprouting be prevented thus assuring that high yielding, quality grain is supplied to the end user.

It is desirable to provide methods for containing cultivated crops within a designated growing area and the prevention of volunteers.

A further object of the invention is to obviate or mitigate the problem of pre-harvest sprouting.

According to the present invention there is provided a method for containing the genetic material of a plant, said method comprising transforming a plant with an expression system functional in a plant and comprising:
(a) an inducible promoter sequence responsive to the presence or absence of an exogenous chemical inducer;
(b) either
   (i) a gene encoding a repressor protein under control of the said inducible promoter,
   or (ii) a gene encoding an inhibitor of the disrupter gene specified at (d) below under control of the said inducible promoter;
(c) a plant developmental gene promoter sequence activated at a selected stage of plant development, which, in the case of (b)(i) above, includes an operator sequence recognised by the said repressor protein, the presence of which inactivates the said plant developmental gene promoter;
(d) a gene encoding a protein-disrupter of a plant-characteristic which characteristic is either essential to plant growth or development or which is a characteristic resulting from gene insertion, under the control of the said plant developmental gene promoter sequence;
whereby the presence or absence of the exogenous chemical inducer controls whether said characteristic is displayed in the plant.

The construct used in the method according to the present invention includes a plant development promoter (PDP) sequence under control of the disrupter protein gene, for restricting expression of the disrupter protein gene to a suitable stage of plant development.

Further the said inducible promoter may promote expression of the repressor protein in response to stimulation by an exogenous chemical inducer whereby in the absence of the chemical inducer no repressor protein is expressed to interact with the operator thus permitting expression of the disrupter protein gene and in the presence of the chemical inducer repressor protein is expressed thereby preventing expression of the gene encoding the inhibitor of plant development permitting unimpeded plant growth.

Alternatively, the said inducible promoter may promote expression of a specific inhibitor of said disrupter protein thereby nullifying the effect of the disrupter protein (for example barstar inhibition of barnase).

Preferably, the plant characteristic controlled by the said expression system is essential to plant growth and the presence of absence of the exogenous chemical inducer allows either growth to maturity or causes growth to slow down or stop at said selected stage.

Preferably, the inducible promoter sequence is functionally linked to and controls a repressor protein gene and in which the disrupter gene promoter includes an operator sequence recognised by the said repressor protein, so that in the presence of the inducer the repressor protein is produced which interacts with the operator sequence disabling the plant developmental gene promoter and inhibiting expression of the disrupter gene.
The disrupter gene, preferably, encodes either (i) a cytotoxin which disrupts cell function, leading to cell death, or (ii) a recombinase or a related enzyme of similar function adapted to excise a nucleotide sequence flanked by recombinase recognition sequences, or (iii) a nucleotide sequence adapted to inhibit an endogenous plant gene which is essential to plant development or an inserted gene conferring a desired characteristic on the plant. The disrupter gene, preferably, encodes a nucleotide sequence adapted to inhibit an endogenous plant gene which is essential to plant development or an inserted gene conferring a desired characteristic on the plant and wherein said nucleotide sequence is in anti sense orientation to the gene to be inhibited and corresponds to less than the full length of the said gene to be inhibited.

Preferably, the gene to be inhibited is an endogenous plant gene essential to seed germination or early seedling development. The gene to be inhibited or excised may be an α-amylase gene.

Preferably, the plant development promoter sequence is the promoter of a gene normally active during germination or early seedling development. Preferably, the promoter is the promoter of the malate synthase gene, the promoter of the germin gene or a promoter selected from the group consisting of the gene promoters of glyoxysomal enzyme genes, aleurone layer genes and carboxypeptidase genes.

According to the method of the present invention the recombinase gene may be the FLP gene of the 2 micron plasmid of Saccharomyces ceravisiae and the recognition sequences may be the FRT sequences which flank all or part of an inserted gene or its regulatory elements.

Alternatively, the disrupter gene may be a recombinase or a related enzyme of similar function adapted to excise a nucleotide sequence flanked by recombinase recognition sequences, and wherein the recombinase gene is the Cre gene of bacteriophage P1 and its recognition sequences are the lox sequences which flank all or part of an inserted gene or its regulatory elements.

Preferably, the disrupter gene is a recombinase or a related enzyme of similar function adapted to excise a nucleotide sequence flanked by recombinase recognition sequences, and wherein the recombinase gene is the Activator transposase of Zea mays.

The inducible promoter is, preferably, the promoter of the gene encoding the 27 kd protein of glutathione-S-transferase II.

Alternatively, the inducible promoter may comprise the promoter of the AlcA gene, the system further comprising a gene capable of expressing the AlcR protein, alcA and alcR being obtainable from Aspergillus.

Preferably, the expression system comprises a repressor protein gene and wherein the repressor protein gene encodes the lac repressor or a repressor used by 434, P22 or lambdabacteriophages.

Alternatively, the expression system comprises a repressor protein gene and wherein the repressor protein is the tet repressor.

According to a further preferred embodiment, the disrupter gene of the expression system encodes barnase and the gene encoding the inhibitor of the disrupter gene contains the coding region of the barstar gene which on expression produces a protein inhibitor of barnase.

According to a second aspect of the present invention there is provided a method for preventing pre-harvest sprouting of a plant, said method comprising transforming a plant with an expression system functional in a plant and comprising:
(a) an inducible promoter sequence responsive to the presence or absence of an exogenous chemical inducer;
(b)
   either (i) a gene encoding a repressor protein under control of the said inducible promoter,
   or (ii) a gene encoding an inhibitor of the disrupter gene specified at (d) below under control of the said inducible promoter;
(c) a plant developmental gene promoter sequence activated at the germination or early seed development stage of a plant, which, in the case of (b)(i) above, includes
(d) an operator sequence recognised by the said repressor protein, the presence of which inactivates the said plant developmental gene promoter;
(e) a gene encoding a protein-disrupter of plant germination, under the control of the said plant developmental gene promoter sequence;
whereby the presence or absence of the exogenous chemical inducer controls whether a seed of the said plant will germinate.

The inducible promoter sequence used in the second aspect of the present invention is, preferably, functionally linked to and controls a repressor protein gene and in which the disrupter gene promoter includes an operator sequence recognised by the said repressor protein, so that in the presence of the inducer the repressor protein is produced which interacts with the operator sequence disabling the plant developmental gene promoter and inhibiting expression of the disrupter gene. In this regard, the disrupter gene encodes a nucleotide sequence adapted to inhibit an endogenous plant gene which is essential to germination. The presence of endogenous chemical will allow the seed to germinate.
Thus the method of the invention leads to a plant which can be reversibly inhibited at an appropriate developmental stage in which said plant contains, stably incorporated in its genome, the recombinant DNA construct defined above.

It is preferred that the inducible promoter promotes expression of the repressor protein in response to stimulation by the exogenous chemical. In the absence of the chemical inducer no repressor protein is expressed to interact with the operator thus permitting expression of the gene encoding the inhibitor of plant development and in the presence of the chemical inducer repressor protein is expressed thereby preventing expression of the gene encoding the inhibitor of plant development allowing the plant to reach maturity. Thus the construct used in the method of the invention contains several operatively linked sequences (a) above will be referred to for convenience as "the chemical switch": (b) as "the repressor sequence": (c) as "the operator" (d) as "the plant development promoter and (e) as "the disrupter gene". The essential elements of each of the sequences and their interaction will be described below with reference to the accompanying drawings.

As an alternative the repressor sequence may be replaced by a gene encoding an inhibitor of the disrupter protein gene. In this situation in the absence of the chemical no inhibitor is produced permitting expression of the disrupter protein, and in the presence of the chemical inducer the inhibitor is produced thereby inhibiting expression.

One example of a gene which is expressed very early in plant development is the malate synthase gene from which its promoter would be suitable for use in this invention. Another example of an early promoter is that associated with the gene which expresses the protein germin.

According to an embodiment of this invention, a chemical switch may be used to control the activity of a recombinase enzyme, or a related enzyme with similar properties. The function of this enzyme is to excise a region of DNA flanked by the terminal repeat sequences recognised by the enzyme as targets for its activity. In this invention the target DNA will be part of an introduced gene, for example, herbicide or insect resistance. Excision of that part of the introduced gene will result in loss of the introduced trait. Activation of the recombinase from an early seedling promoter or other plant development promoter is controlled by a chemical switch and repressor/operator system as described above.

An advantage of the this embodiment is that the parent line can easily be maintained but in the absence of the chemical needed to induce the chemical switch the value-added trait, such as herbicide or insect resistance, is lost.

A first example of the recombinase is the FLP gene from the 2 micron plasmid of Saccharomyces cerevisiae. The terminal repeat sequences (FRT) required for recombination have been described in the literature (Gronoskijiski and Sadowski, 1985, Journal of Biological Chemistry, 260, 1230-1237: Senecoff et.al., 1985, Proc. Natl. Acad. Sci. USA, 82, 7270-7274). The nucleotide sequence encoding the FLP gene have also been described (nucleotides 5568 to 6318 and 1 to 626 of the 2-micron plasmid displayed by Hartley and Donelson, 1980, Nature, 286, 860-865). The use of FLP and its FRT repeats to excise gene sequences has been shown for mammalian (O'Gorman et.al., 1991, Science, 251, 1351-1355) and plant cells (Lyznik et.al., 1993, Nucleic Acids Research, 21, 969-975). The general use of the FLP recombinase is the subject of International Patent Application Number WO 92/15694.

A second example of the recombinase is the CRE recombinase of bacteriophage P1 (Hoess and Ambremski, 1985, J.Mol.Biol. 181, 351-362) which recognises the lox repeat recombination system. The use of the Cre-lox system to promote site specific recombination has been demonstrated in mammalian (Sauer and Henderson, 1988, Proc. Natl. Acad. Sci. USA, 85, 5166-5170) and plant cells (Dale and Ow, 1990, Gene, 91, 79-85; Russell et.al., 1992, Mol. Gen. Genet., 234, 49-59). The general use of the Cre-lox system is the subject of European Patent Application Number 220,009.

A third example of the recombinase gene is that encoded by the Activator (Ac) transposase element from maize. The terminal repeat sequences which flank target regions have been described in the literature (Pohlman et.al. 1984, Cell 37, 635-643) and the nucleotide and amino acid sequences of the transposase gene have been reported (Kuze et.al, 1987, EMBO J., 6, 1555-1563). The Activator transposase causes excision of itself and other regions of maize and other plants as described in Dean et.al. (The Plant Journal, 2, 69-81, 1992) and references therein.

Both aspects of the system are inherited as Mendelian characteristics. This will be achieved through the insertion into the plant genome of the molecular elements required for the control of plant growth through to maturity.

This invention enables the production of plant varieties which are rendered non-viable during growth and development such that full-sized seed is not produced, or which are inhibited from attaining their full development potential or full expression of all the genetically encoded traits or which are prevented at the seed stage from germinating. These plants require a chemical switch system for the reversal of the disruption effect so that the plants can grow to maturity and set seed.

This invention can be used for the protection of the germplasm of any mono- or di-cotyledonous inbred lines which may be sold as inbreds or as hybrids and for which suitable transformation techniques are or become available, particularly maize, wheat and other small grain cereals, sunflower, oil seed rape, soybeans, tomato and other vegetables, sugar beet and ornamental foliage and flowering plants.

The system used in the invention will be readily transferable between lines and into new crop species. Full growth in the seed producer's, breeder's and farmer's field will be ensured by simple application of a chemical to the seed coat or to the developing plant.

In one specific application we describe the production of plants particularly inbred plants, which are inhibited during the early stages of seedling growth using molecular engineering techniques. These plants can be reversed to full growth capability by application of a chemical to the seed coat post harvest but before planting which, upon germination of the seed, leads to a molecular control cascade which relieves inhibition of early seedling growth and permits growth to maturity and setting of seed.

### 1. BRIEF DECRIPTION OF THE DRAWINGS

Figures 1 and 2 are schematic illustrations of the gene action which occurs within the recombinant gene used in the invention in the absence (Figure 1) or the presence (Figure 2) of the chemical inducer;
Figure 3 is an illustration of a second embodiment of the invention using recombinases to effect gene excision in the absence of the inducer;
Figure 4 gives the nucleotide sequences of various primers used in Example 1 hereinafter;
Figure 5 gives the sequence of the PCR primers used to amplify the barnase and barstar genes;
Figure 6 summarises the cloning strategy for barnase and barstar;
Figure 7 is a map of plasmid pJR1Ri;
Figure 8 is a map of plasmid pPOP1;
Figure 9 illustrates the structure of vectors pTAK1, pTAK2 and pTAK3;
Figure 10 is a graph of the levels of GUS expression for two PCR-positive plants generated as described in Example 3; and,
Figure 11 is a map of the plasmid pSWE1.

### 2. THE OVERALL PROCESS

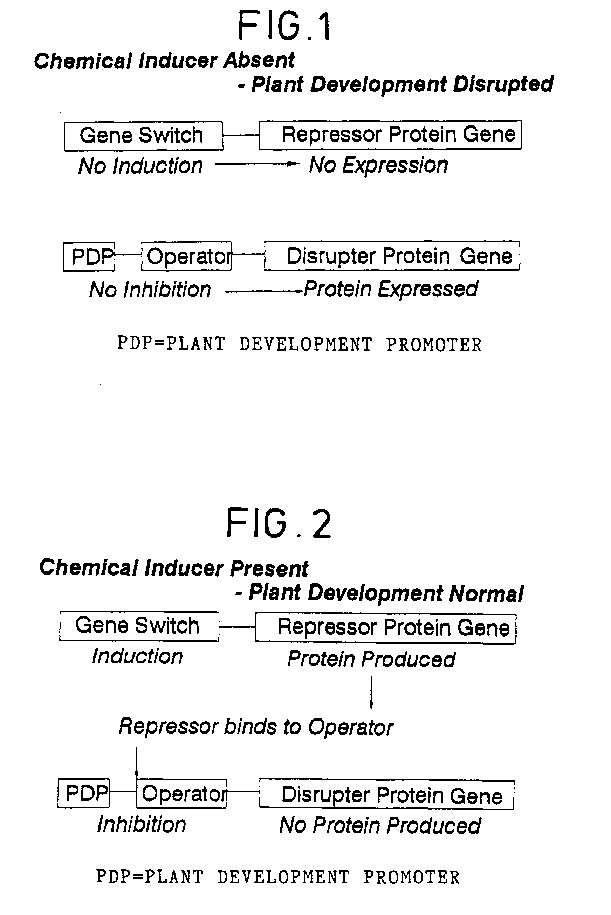

Figure 1 of the drawings is a block diagram of the DNA construct used in the invention in the growth inhibited state. In the absence of the exogenous chemical inducer, the chemical switch is inactive and no repressor protein is expressed by the repressor sequence. In the absence of the repressor protein, the operator sequence permits expression of the disrupter protein during plant development such that growth to maturity and pollination of seed is inhibited. In one specific embodiment early growth of seedlings may be inhibited by expression of the disrupter gene being specifically directed to these stages of development by an early seedling growth (ESG) promoter sequence. The outcome being that the plant fails to reach maturity and set full sized seed.

Figure 2 shows the operation of the construct in the "growth permitted" state. When the chemical inducer is brought into contact with the plant, the chemical switch is activated causing the repressor protein to be expressed. The repressor protein then binds the operator, inhibiting expression of the disrupter protein and restoring growth to maturity.

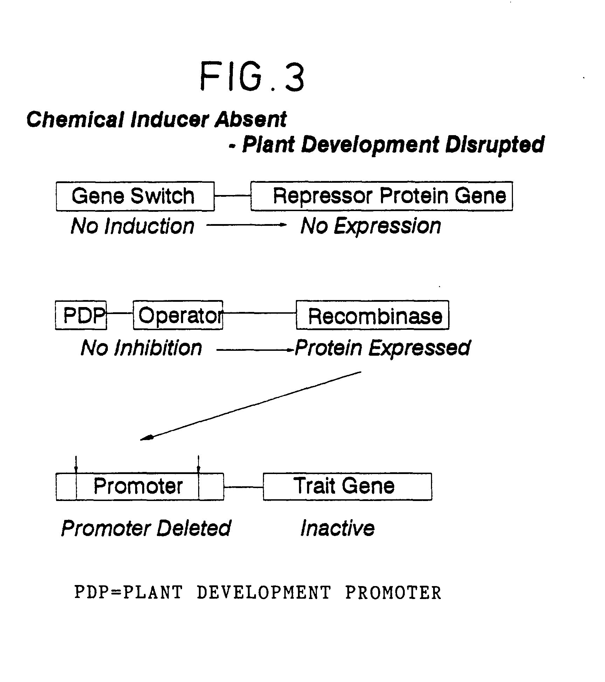

Figure 3 illustrates a second embodiment of the present invention using a transposase to inhibit the action of a promoter against which it is directed.

### 3. THE CHEMICAL SWITCH

One form of chemical switch is the subject of our International Patent Applications No. WO 90/08826 (published 9th August 1990) and WO 93/01294 (published 21st January 1993).

A large number of plant promoters are assumed to be induced using chemical signals. However, it has only been demonstrated in few examples that the specific chemicals switch on gene expression in the tissues required for this invention. The gene of particular interest is the gene encoding the 27kd subunit of glutathione-S-transferase II (GSTII). (See WO 90/08826.) The full sequence of the promoter of that gene is the given in WO 93/01294. This gene is induced specifically upon treatment of plant tissues using chemical safeners. One such safener is N,N,- diallyl-2,2- dichloroacetamide, but there are related compounds which have improved mobility characteristics in plants tissues, combined with improved persistence for this application, efficacy and safety. These compounds have been described in the literature.

It is obvious that additional chemically induced promoters can be used in this scenario. Some of these may be of plant origin, others may be of fungal, bacterial or yeast origin. It is implied in the present application that those promoters and chemical combinations suitable for the plant growth control procedure can be used in place of GSTII and safeners.

An additional example is the alcR activator gene and the alcA target promoter from Aspergillus. The chemical inducer is cyclohexanone. The alcA gene promoter is an inducible promoter, activated by the alcR regulator protein in the presence of inducer (ie by the protein/alcohol or protein/ketone combination). The alcR and alcA genes (including the respective promoters) have been cloned and sequenced (Lockington RA et al, 1985, Gene, 33:137-149; Felenbok B et al, 1988, Gene, 73:385-396; Gwynne et al, 1987, Gene, 51:205-216).

Alcohol dehydrogenase (adh) genes have been investigated in certain plant species. In maize and other cereals they are switched on by anaerobic conditions. The promoter region of adh genes from maize contains a 300 bp regulatory element necessary for expression under anaerobic conditions. However, no equivalent to the alcR regulator protein has been found in any plant. Hence the alcR/alcA type of gene regulator system is not known in plants. Constitutive expression of alcR in plant cells does not result in the activation of endogenous adh activity.

Another example of a chemically inducible gene is given in European Patent Application EP-A-0332104 (Ciba-Geigy).

### 4. THE REPRESSOR AND OPERATOR SEQUENCES

One such operator/repressor system is the subject of our published International Patent Application No. WO 90/08829 (published 9th August 1990).

In a first embodiment we propose to use the well-characterised interaction between bacterial operators with their repressors to control the expression of the disrupter gene function. Bacterial repressors, particularly the lac repressor, or repressors used by 434, P22 and lambda bacteriophages can be used to control the expression in plant cells very effectively.

Another example of an operator/repressor system is the tet (tetracycline) repressor and target operator, the inducer being tetracycline (see, for example, Gatz et.al., 1991, Mol. Gen. Genet., 227, 229-237)

A second operator/repressor system is the subject of our published International Patent Application No. WO 90/08827 (published 9th August 1990).

In a second embodiment it is possible to utilise 'pseudo-operators', operators which are similar but not identical to the normally used operators in a particular operator-repressor combination. We have demonstrated that using a suitable selection system mutant repressors can be generated which recognise pseudo-operators found in plant genes. We describe below the selection of mutant repressors recognising pseudo-operators which are found in plant genes.

A third approach for the down-regulation of the disrupter genes which can be considered is the use of either antisense RNA or partial sense RNA. Both of these approaches have been demonstrated to work well for the regulation of polygalacturonase expressed during tomato fruit ripening (Smith et.al., 1988, Nature, 334, 724-726; Smith et.al., Mol. Gen. Genet., 1990, 224, 477-481).

A fourth approach to the inhibition of disrupter genes which can be used is the use of specific inhibitors of the disrupter protein as this has been demonstrated in male sterile plants which have been rendered sterile by the activity of a barnase gene and which can be restored to fertility by the action of a barnase inhibitor, barstar (Mariani et.al., Nature 6377, p384, 1992)

### 5. PLANT DEVELOPMENT PROMOTER

As already described, expression of the disrupter genes should be directed to stages of plant growth, which if inhibited, would prevent the plant reaching full maturity and setting seed.

Particular examples of suitable stages for inhibition would be the very early stages of seedling growth shortly after germination or during development of the flower which gives rise to the fruit containing seed or to the fruit itself where the term fruit is used in its widest sense to describe the organ containing seed. One example of a gene which is activated very early in development is the malate synthase gene, the promoter of which is suitable (see Graham et.al., 1990, Plant Mol. Biol., 15, 539-549; Comai et.al., 1992, Plant Physiol., 98, 53-61).

Further examples of plant development promoters are from the genes in the glyoxysome such as isocitrate lyase, and, promoters from genes in the aleurone layer such as α-amylases (Baulcombe et.al., (1987) Mol. & Gen. Genet. 209, 33-40, and references therein). One may also use scutellum gene promoters such as that of carboxypeptidase. Another possibility is a promoters from germin genes (Lane et.al. (1991) J.Biol.Chem. 266, 10461)

DNA promoter sequences which drive the expression of genes at the appropriate growth stages of which several examples are given above can be achieved using established protocols for the identification of genes expressed in specified organs or tissues through differential screening of cDNA libraries cloned in various vectors systems, the isolation of genes encoding these cDNAs from genomic libraries using bacteriophage lambda vectors, and the characterisation of their promoter sequences using DNA sequencing and analytical plant transformation experiments.

### 6. DISRUPTER GENE

Inhibition of plant growth will be achieved by using novel disrupter genes which, when expressed specifically at a suitable stage of plant development (for details see above), will lead inhibition of growth and development such that plants fail to reach maturity and to set seed.

Disrupter genes are described in our published International Patent Application No. WO 90/08831 (published 9th August 1990).

The origin of the disrupter genes can be from a variety of naturally occurring sources, e.g. human cells, bacterial cells, yeast cells, plant cells, fungal cells, or they can be totally synthetic genes which may be composed of DNA sequences some of which are found in nature, some of which are not normally found in nature or a mixture of both. The disrupter genes will have preferably an effect on mitochondrial metabolism, as it has been quite clearly demonstrated that ample energy supply is an absolute requirement for growth particularly during early seedling development and flowering and fruit formation. However, it is also envisaged that the disrupter function can be effectively targeted to other essential biochemical functions such as DNA and RNA metabolism, protein synthesis, and other metabolic pathways. Two such DNA constructs consist of those sequences encoding the mammalian brown adipose tissue uncoupling protein or variants thereof, or a synthetic gene which consists of a mitochondrial targeting domain, and a lipophilic domain which allows insertion of the protein into the mitochondrial membrane.

Additional examples of suitable disrupter genes are barnase/Ti ribonuclease (Mariani et.al. (1990) Nature 6295, 737)

Of the recombinases, the best known are the Ac transposase of maize, the FLP recombinase from yeast and the Cre recombinase of bacteriophage P1.

### 7. PRODUCTION OF AN EXPRESSION MODULE CONSISTING OF PROMOTER SEQUENCES TARGETING EXPRESSION OF A DISRUPTER GENE TO AN ESSENTIAL STAGE OF PLANT GROWTH

Production of an expression module which consists of the developmental stage of the promoter sequences and the disrupter genes will be done using established molecular techniques. The expression of this module in elite inbreds will lead to the production of the disrupter gene product during an essential stage of growth will result in plants that fail to reach maturity and set seed.

### 8. TRANSFORMATION

Transgenic plants are obtained by insertion of the constructs described into the genome of the plants. The specific transformation procedure employed for insertion of the gene constructs of this invention into the plant genome is not particularly germane to this invention. Numerous procedures are known from the literature such as agroinfection using Agrobacterium tumefaciens or its Ti plasmid, electroporation, microinjection of plant cells and protoplasts, microprojectile transformation and pollen tube transformation, to mention but a few. Reference may be made to the literature for full details of the known methods.

### 9. REVERSAL OF GROWTH INHIBITION

It is apparent, the plants which are made inhibited during growth using the above techniques and methods are not desirable per se. Therefore we proposed to use a cascade using molecular elements which will allow the reversal of the growth inhibition thus permitting growth through to maturity and setting of seed as per normal without any effect on quality or yield of seed.

### 10. DESIGN OF THE REVERSAL MECHANISM.

The reversal mechanism proposed here consists of three separate elements:
a. a chemically switchable promoter.
b. a bacterial operator sequence.
c. a bacterial repressor gene which binds with high affinity the aforementioned operator.

These elements will act in the following way:-

When restoration of growth is required the plants are treated with the chemical at an appropriate state. In a specific embodiment of this process growth is inhibited during the very early stages of seedling development, shortly after germination of the seed. There the chemical would preferably be applied as a coating to the seed prior to sowing.

This chemical induces through a chemically-inducible promoter the expression of a bacterial repressor molecule which will bind to operator DNA sequences in the plant growth promoter sequences. This binding will lead to the inhibition of the disrupter gene function, thus allowing normal growth and development to occur and the plants to reach maturity and to set normal seed.

### 11. APPLICATION TO THE CONTAINMENT OF GERMPLASM

Figures 1 and 2 outline the molecular events which will take place when this system is introduced into inbred lines.

The introduced gene cassettes will act as a single dominant genetic locus and will be present in inbred lines in a homozygous condition ensuring that the trait is transferred to all offspring.

This system will enable the seed producer to control the development of any plants containing said system through to maturity and seed set by the simple application of a chemical. The seed produced which is subsequently sold to the farmer will also require application of the chemical before a seed crop can be harvested. In a specific embodiment of this system, growth is inhibited at the early stages of seedling development. In this case the seed produced and sold to the farmer will likely have a seed treatment with the chemical which overcomes the growth inhibition allowing the plants to reach maturity and produce the fruit and/or seed crop.

In subsequent generations of the crop or after outcrossing to related wild species, none of the plants containing the gene construct will grow past the early stages of seedling development thereby providing a means of containing cultivated plants within a designated cultivation area an prevention of volunteers.

### 12. FIRST SPECIFIC EMBODIMENT OF THE SYSTEM

as a specific example of the system of the invention the following components are be used.
a. The chemically suitable promoter element isolated from the maize glutathione-S-transferase gene, which encodes the 27kd subunit of isoform II of the enzyme (GSTII-27).
b. The lac I repressor gene from E.coli.
c. The malate synthase gene promoter from Cucumis sativum which gene expression to the very early stages of seedling development immediately post-germination.
d. The lac operator sequence incorporated as a replacement in the malate synthase promoter between the TATA-element and the transcription start point.
e. The mammalian uncoupling protein (UCP) isolated from the brown adipose tissue of Ratus ratus, which inhibits growth of plant cells by uncoupling of mitochondrial respiration.
f. Chemical inducers of the GSTII-27 promoter, namely the herbicide safeners R-25788 and R-29148, these chemicals effecting the reversal of the growth inhibition.

### 13. THE PLASMIDS

A plasmid p35SlacI containing the lacI gene repressor/operator has been deposited in an E.coli strain TG-2 host at the National Collection of Industrial & Marine Bacteria in Aberdeen, UK, on 12th December 1988, under the Accession Number NCIB 40092.

A plasmid containing genomic DNA which includes the promoter sequence and part of the GSTII enzyme was deposited on 14 June 1991 in the National Collections of Industrial and Marine Bacteria (NCIMB), 23 St Machar Drive, Aberdeen, AB2 1RY, UK, as plasmid pGIE7 contained within Escherichia coli, strain XLI-Blue with the Accession Number NCIMB 40426.

The following specific Examples illustrate the invention.

### Example 1

### PCT AMPLIFICATION AND INSERTION OF A lac I OPERATOR SEQUENCE INTO THE MALATE SYNTHASE PROMOTER.

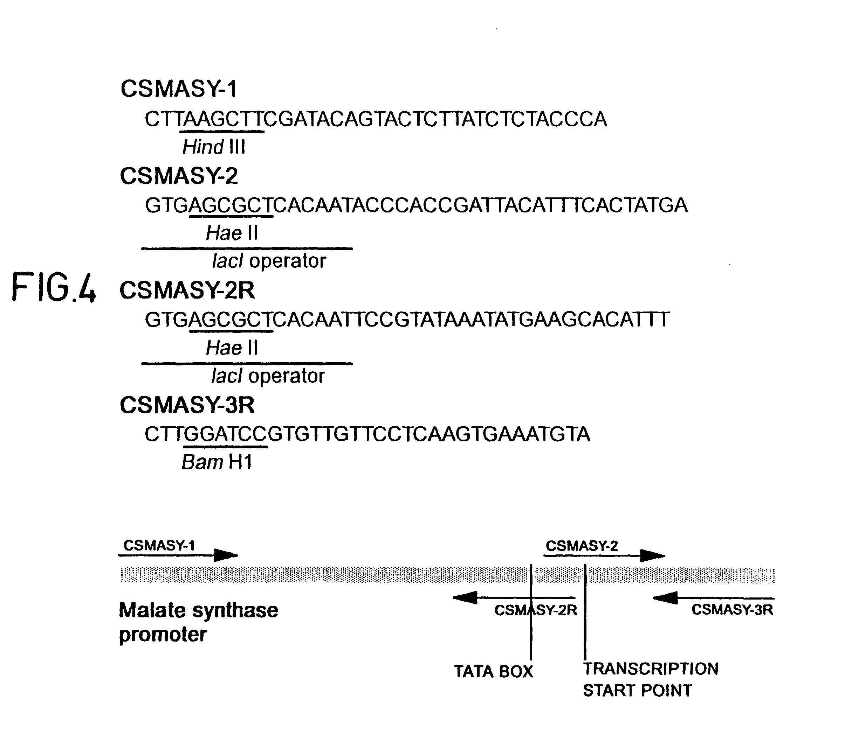

The PCR was used to amplify the malate synthase promoter fragment from cucumber using the sequence information published previously (Graham et.al., 1989, Plat Mol. Biol., 13, 673-684). Three primer pairs were used. CSMASY-1 with CSMASY-3R produce a promoter fragment containing 1886 base pairs of sequence upstream from the translation start point. CSMASY-1 with CSMASY-2R and CSMASY-2 with CSMASY-3R produce two promoter fragments of 1847 and 55 base pairs respectively which introduce by nucleotide substitution a consensus lac I operator sequence between the transcription start point and the TATA box when cut with HaeII and religated. (see WO 90/08829) Figure 4 shows the sequence of the four primers and the strategy for amplification of an unmodified and modified (operator inserted) versions of the malate synthase promoter.

The unmodified promoter fragments were subcloned into the polylinker of pUC19 following digestion with HindIII and BamHI to provide compatible cohesive ends. The operator modified version of the malate synthase promoter was constructed in a three-way ligation between the 1847 base pair and the 55 base pair PCR products and pUC 19 following digestion with BamHI and HindIII (pUC 19 and PCR products) and HaeII (PCR products only). Following transformation of E.coli and preparation of plasmid DNAs, the sequence of the unmodified (pMSl) and the modified (pMSOP1) PCR-amplified malate synthase promoter was checked by dideoxy sequence reactions.

### Example 2

### CONSTRUCTION OF A PLANT TRANSFORMATION VECTOR pPOP1 AND TRANSFORMATION OF TOBACCO.

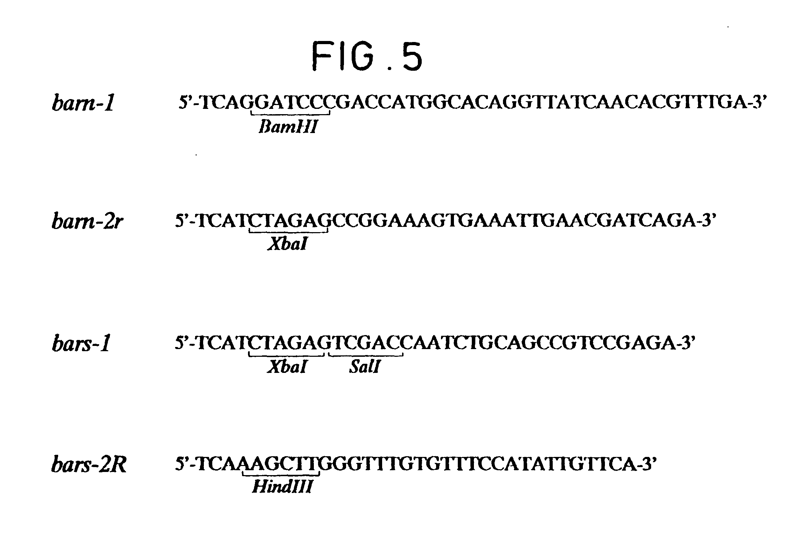

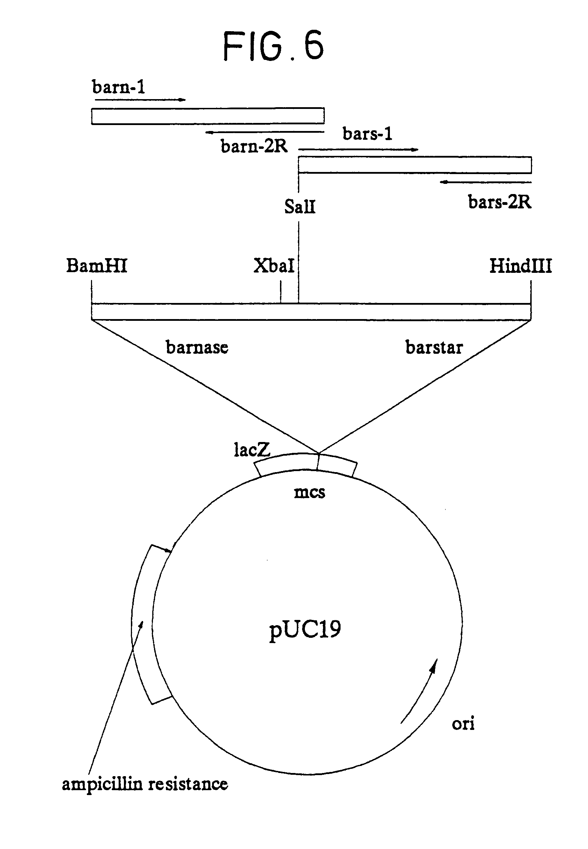

The unmodified malate synthase promoter of pMSl was fused to the barnase gene from Bacillus amyloliquefaciens. The barnase gene was introduced as a blunt-ended 0.9Kb fragment into the BamHI cut, filled in linear pMSl plasmid thus positioning the barnase open reading frame immediately downstream of the malate synthase promoter and untranslated leader sequence, producing plasmid pMSB1. The barnase gene was amplified from Bacillus amyloliquefaciens DNA using sequence information described by Hartley, 1988, J. Mol. Biol., 202, 913-915). The barnase cassette also contains the barstar gene which encodes a specific proteinaceous inhibitor of barnase. The barstar gene is arranged such that it is not in the same reading frame as barnase but is fused to a promoter active in E.coli. The presence of the barstar gene facilitates the manipulation of the barnase gene in E.coli where low levels of unprogrammed expression may be lethal to the bacterial cells. This strategy is again described by Hartley (see above) and the references therein. The sequence of the PCR primers used to amplify the barnase and barstar gene is shown in Figure 5 and the cloning strategy in Figure 6.

Following introduction of the barnase gene cassette and orientation such that the translation start codon was in-frame with the malate synthase promoter the nopaline synthase (nos) 3' polyA addition sequence was introduced into pMSB1 at the distal end of the barnase cassette as a KpnI/EcoRI fragment from pIE98, producing pMSBN1. A synthetic linker (RNOT-1/2) comprising oligonucleotides RNOT-1 (5'-AATTGCGGCCGCATTATG-3') and RNOT-2 (5'-AATTCATAATGCGGCCGC-3') was then introduced at the EcoRI site of pMSBN1. This linker provides a unique NotI site within the pMSBN1 plasmid, destroys one flanking EcoRI site and retains the other. The linker insertion is orientated such that the remaining EcoRI site flanks the full cassette and is distal to the NotI site. An inducible barstar cassette was then introduced as an EagI fragment into the NotI site of the linker adapted pMSBN1 plasmid to produce plasmid pMSBNIB1. The sequence flanking the EagI insert are such that one NotI site is recreated by the ligation. The inducible barstar cassette comprises the barstar gene from B.amyloliquefaciens flanked, and its expression controlled, by a 0.9Kb fragment of the safener-inducible GSTII-27 gene and the 300 bp nos 3' polyadenylation sequence.

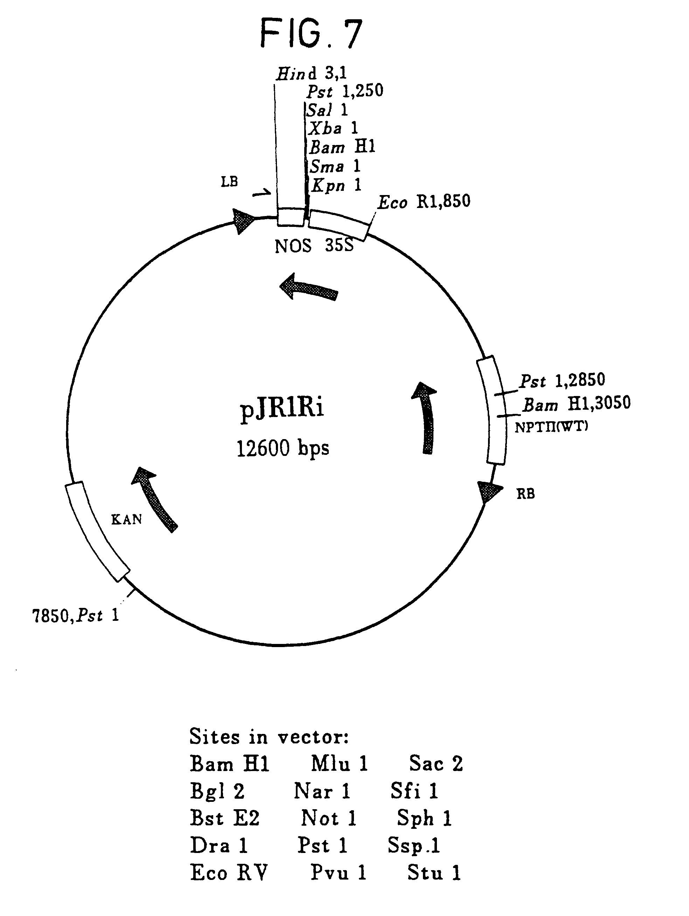

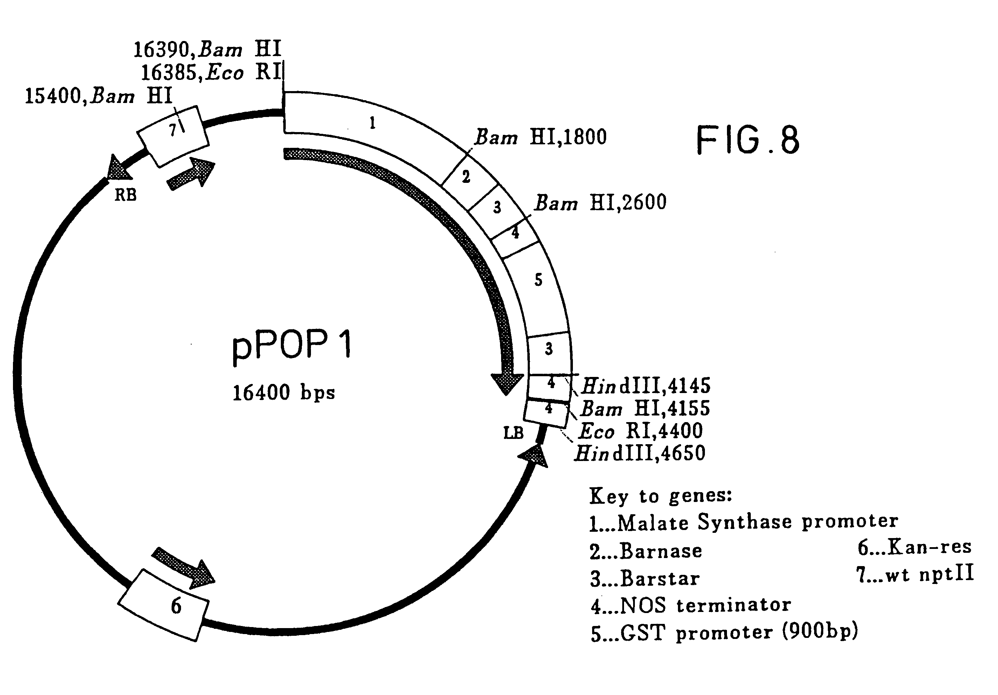

Finally, the full cassette of pMSBNIB1 was transferred to the plant transformation vector pJRIR1 (Figure 7) as an EcoRI/ partial HindIII blunt-ended fragment and ligated into the XbaI cut, phosphatased blunt-ended pJRIR1 vector to produce the plasmid pPOP1 (Figure 8). The plasmid pPOP1 was used to transform the disarmed Agrobacterium tumefaciens strain LBA4404 (pAL4404)(Hoekema et.al., 1983, Nature, 303, 179-180) using the freeze-thaw method. A single colony was grown up in 40 ml of LB medium at 28°C in an orbital incubator at 200 revs/min overnight until the culture reaches an O.D.₅₈₀ of 0.5-1.0. The cells were resuspended in 1 ml of ice cold 20 mM CaCl₂ solution, and then dispensed into prechilled eppendorfs, 100 µl per tube. DNA was added to the cells, 0.1 µg/100 µl of cells, and then the cells were frozen in liquid nitrogen. The cells were then thawed at 37°C in a water bath for 5 minutes. 1 ml of LB medium was added to each tube and the cells were incubated at 28°C for 2-4 hours with gentle shaking to allow the bacteria to express the antibiotic resistance genes. The cells were centrifuged for 30 seconds at 13,000 revs/min in a microcentrifuge and the supernatant was discarded. The cells were resuspended in 100 µl of LB medium. The cells were spread onto LA agar plates containing 50 µg/ml kanamycin, or other antibiotic selection afforded by the introduced plasmid. The plates were incubated at 28°C for 2 days, when colonies were likely to appear.

### Plasmid minipreps from Agrobacterium tumefaciens

Single colonies were grown in 20 ml of LB medium containing antibiotic as the selective agent overnight at 28°C in an orbital incubator. The cells were centrifuged at 3000 revs/min for 5 minutes and the pellet was resuspended in 0.5 ml of miniprep solution, (5 mg/ml lysozyme in 50 mM glucose, 10 mM EDTA, 25 mM Tris-HCl pH 8.0). The cells were incubated on ice for an hour, and then 1 ml of alkaline SDS (0.2 M NaOH, 1% SDS) was added. After incubation on ice for 10 minutes, 0.75 ml of 3 M sodium acetate was added, and the mixture was left on ice for 30 minutes. The lysis mixture was centrifuged at 15,000 revs/min for 10 minutes at 4°C, and the supernatant was transferred to 15 ml corex tubes. 5 ml of cold ethanol was added and the tubes were stored at -70°C for 30 minutes. The tubes were then centrifuged at 15,000 revs/min for 15 minutes at 4°C and the supernatant was removed. The pellet was dissolved in 0.5 ml of T.E. and transferred to eppendorfs. The DNA solution was extracted with an equal volume of phenol/chloroform three times and once with an equal volume of chloroform. The DNA was precipitated by adding 1 ml of ethanol and incubating at -70°C for 30 minutes. After centrifugation at 13,000 revs/min for 15 minutes in a microcentrifuge the DNA pellet was washed with 70% ethanol, dried at room temperature for a few minutes, and redissolved in 50µl T.E. 20µl of DNA solution was used per restriction digest. Generally, a 2-3 fold increase in restriction enzyme and at least 4 hours digestion was required for the Agrobacterium DNA to be digested adequately.

### Tobacco transformations

Transgenic tobacco was generated by the leaf disc method using transformed Agrobacterium tumefaciens. About 20 tissue culture grown plates were required per transformation. The plants were about 3-4 weeks old and were grown on M.S medium without antibiotics. All manipulations were carried out in sterile hoods using sterile implements.

Leaves were cut from the tissue culture plants, placed on NBM medium in petri dishes, and incubated overnight in a plant growth room. The transformed Agrobacterium strain was grown up overnight in 100ml of LB containing kanamycin at 50 µg/ml. The next day the culture was centrifuged at 3000 revs/min for 10 minutes and resuspended in an equal volume of MS solution. 20 ml of Agrobacterium solution was placed in 9 cm petri dishes. Leaf discs were made from the leaves, using a sterile scalpel, and were put into the Agrobacterium solution in the petri dishes for 20 minutes. The leaf pieces were then transferred to the NBM plates and incubated overnight in a plant growth room. After 48 hours the leaf discs were transferred to NBM medium containing carbenicillin at 500 µg/ml and kanamycin at 100 µg/ml in neoplant pots. The pots were incubated in a plant growth room for 4-6 weeks. Shoots emerging from callous tissue were transferred to MS medium containing carbenicillin at 200 µg/ml and kanamycin at 100 µg/ml in neoplant pots, (7 to a pot). After 3 weeks, shoots that had rooted were transferred to fresh MS medium and grown on until they were about 5 cm in height. Extra cuttings were taken at this stage. The plants were then transferred to compost in 13 cm pots and sealed in polythene bags to prevent dehydration for the first few weeks.

After transfer of the plantlets to the greenhouse, PCRs were performed on leaf samples to check for the presence of the malate synthase/barnase and GSTII-27/barstar gene fusions and 31 plants containing both gene cassettes grown to maturity.

After flowering of the plants and a backcross with wild-type pollen, seed are collected and germinated on moist filter paper containing water alone or water plus 30 ppm of the safener chemical, dichlormid,. In the absence of safener and in plants containing a single insertion site, approximately 50% of the seed are expected to germinate. In the presence of the safener, 90-100% of the transgenic seed will germinate. PCR analysis of the seedlings growing in the absence of safener will show that these plants are azygous for the POP1 vector. Similarly PCR will show that approximately 50% of seedlings grown in the presence of safener contain the POP1 vector (i.e. are homozygous or heterozygous for the introduced genes).

This shows that the safener inducible barstar gene can be used to overcome the deleterious effects of barnase produced from the malate synthase promoter following seed germination.

### Example 3

### DEMONSTRATION THAT A MODIFIED MALATE SYNTHASE PROMOTER CONTAINING THE lacI OPERATOR SEQUENCE TARGETS GENE EXPRESSION TO GERMINATING SEEDLINGS.

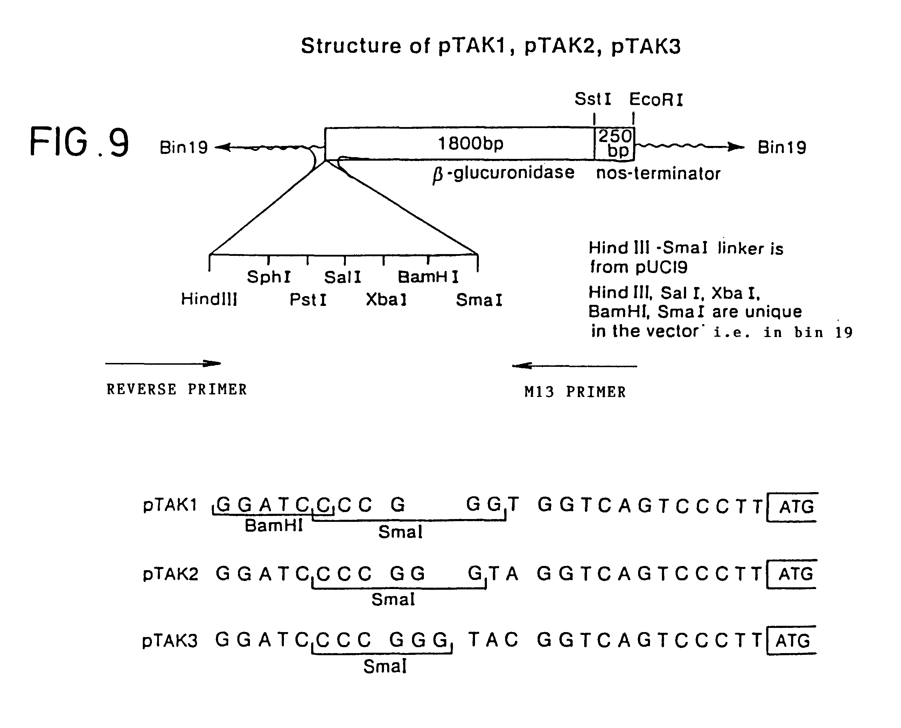

The modified malate synthase promoter from pMSOP1 was transferred to the plant transformation vector pTAK1 (Figure 9). The 1800 bp modified malate synthase promoter was transferred as a HindlII/BamHI fragment and introduced into pTAK1 such that the promoter is fused to the glucuronidase (GUS) gene in pTAK1.

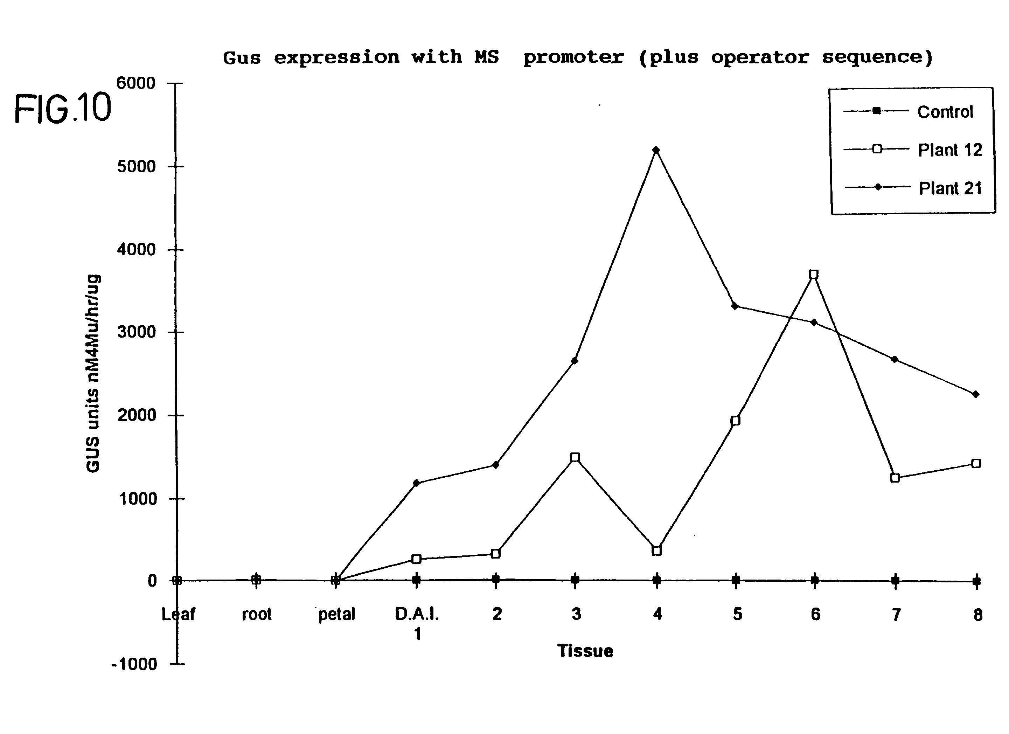

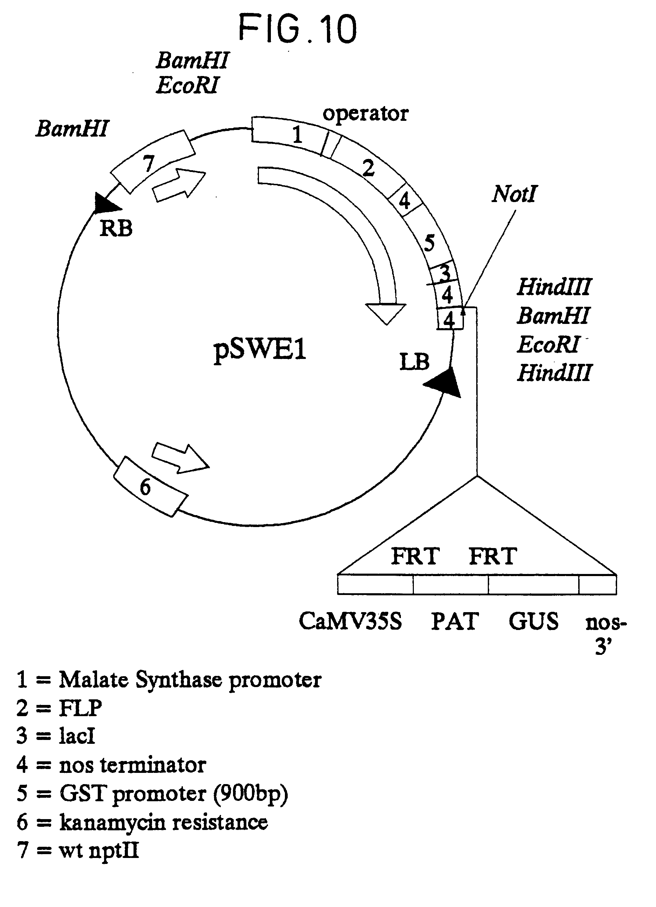

Following transformation of Agrobacterium tumefaciens and generation of transgenic tobacco plants as described in Example 2 above, the various parts of PCR-positive plants were assayed for GUS activity. In addition, germinating seedlings from self-pollinated plants were harvested at various days after imbibition (DAI). The data from two such plants and a control are shown in Figure 10 Note that the promoter activity as measured in GUS units is only detected in seedlings following imbibition. Note also that the seedling assays were done on self-pollinated seed containing homozygous, heterozygous and azygous progeny.

### Example 4

### DEMONSTRATION OF FLP MEDIATED EXCISION OF A FRT-FLANKED PAT GENE AND SUPPRESSION OF EFFECT BY AN INDUCIBLE REPRESSOR GENE.

The POP1 vector described in Example 2, but containing the modified malate synthase promoter with operator sequence, was adjusted such that the barnase gene was replaced by the 1.5 Kb FLP coding sequence from plasmid pOG44 (purchased from Stratagene, La Jolla, California) and the barstar gene fused to the GSTII-27 was replaced by the lacI coding sequence (see WO 90/08829). This new vector is designated pSWE1 (Figure 11).

To provide an assay of FLP activity and a model for the switchable removal of a trait gene, a gene fusion between the CaMV35S promoter, the PAT (phosphinothricin-acetyl transferase) gene for resistance to the herbicide bialaphos and glucuronidase (GUS) was constructed.

The construct is arranged such that the PAT gene, flanked by FRT recombination sites, interrupts the expression of GUS from the CaMV35S promoter. Thus, excision of PAT by FLP-mediated recombination, will activate expression of GUS thus providing an assay for excision. Prior to excision the PAT gene is expressed by the CaMV35S promoter thus providing a scorable phenotype for lack of recombination, particularly during the switched suppression of FLP activity by the repressor protein. The complete transcription unit so described is terminated by the nos 3' polyadenylation sequence. This scorable cassette is introduced into a unique NotI site in the SWE1 vector described above.

The construct described above is transformed into Agrobacterium tumefaciens and transgenic tobacco plants prepared as described in Example 2. Following a backcross with wild-type pollen the plantlets may be tested for the activation of GUS in the transgenic individuals grown in the absence of safener. Plants with a single site of insertion, germinated on moist filter paper containing water alone, will show that 50% of these seedlings will have GUS activity and increased sensitivity to bialaphos. Excision of the PAT gene in these seedlings may be confirmed by Southern blotting. When seedlings are germinated on 30 ppm of the safener dichlormid, all the seedlings will show no activation of GUS expression and will retain resistance to bialaphos.

This demonstration shows that a recombinase enzyme can be used to excise a gene in transgenic plant cells and that a chemically regulated repressor gene can suppress the effects of the recombinase by its action on an operator sequence introduced into the promoter controlling the expression of the recombinase gene.

## Claims

1. A method for containing the genetic material of a plant, said method comprising transforming a plant with an expression system functional in a plant and comprising:
(a) an inducible promoter sequence responsive to the presence or absence of an exogenous chemical inducer;
(b)
either (i) a gene encoding a repressor protein under control of the said inducible promoter,
or (ii) a gene encoding an inhibitor of the disrupter gene specified at (d) below under control of the said inducible promoter;
(c) a plant developmental gene promoter sequence activated at a selected stage of plant development, which, in the case of (b)(i) above, includes an operator sequence recognised by the said repressor protein, the presence of which inactivates the said plant developmental gene promoter;
(d) a gene encoding a protein-disrupter of a plant-characteristic which characteristic is either essential to plant growth or development or which is a characteristic resulting from gene insertion, under the control of the said plant developmental gene promoter sequence;
whereby the presence or absence of the exogenous chemical inducer controls whether said characteristic is displayed in the plant.

2. A method according to claim 1 wherein the plant characteristic controlled by the said expression system is essential to plant growth, whereby the presence of absence of the exogenous chemical inducer allows either growth to maturity or causes growth to slow down or stop at said selected stage.

3. A method as claimed in claim 1 or claim 2 wherein the said inducible promoter sequence is functionally linked to and controls a repressor protein gene and in which the disrupter gene promoter includes an operator sequence recognised by the said repressor protein, so that in the presence of the inducer the repressor protein is produced which interacts with the operator sequence disabling the plant developmental gene promoter and inhibiting expression of the disrupter gene.

4. A method as claimed in any one of claims 1 to 3, wherein the disrupter gene encodes either (i) a cytotoxin which disrupts cell function, leading to cell death, or (ii) a recombinase or a related enzyme of similar function adapted to excise a nucleotide sequence flanked by recombinase recognition sequences, or (iii) a nucleotide sequence adapted to inhibit an endogenous plant gene which is essential to plant development or an inserted gene conferring a desired characteristic on the plant.

5. A method according to claim 4 wherein the disrupter gene encodes a nucleotide sequence adapted to inhibit an endogenous plant gene which is essential to plant development or an inserted gene conferring a desired characteristic on the plant and wherein said nucleotide sequence is in antisense orientation to the gene to be inhibited and corresponds to less than the full length of the said gene to be inhibited.

6. A method as claimed in claim 5 in which the gene to be inhibited is an endogenous plant gene essential to seed germination or early seedling development.

7. A method as claimed in claim 6, wherein the gene to be inhibited or excised is an α-amylase gene.

8. A method as claimed in claim any one of claims 1 to 7 in which the said plant development promoter sequence is the promoter of a gene normally active during germination or early seedling development.

9. A method as claimed in claim 8, wherein the said promoter is the promoter of the malate synthase gene.

10. A method as claimed in claim 8, wherein the said promoter is the promoter of the germin gene.

11. A method as claimed in claim 8, wherein the said promoter is selected from the group consisting of the gene promoters of glyoxysomal enzyme genes, aleurone layer genes and carboxypeptidase genes.

12. A method as claimed in claim 4, in which the recombinase gene is the FLP gene of the 2 micron plasmid of Saccharomyces ceravisiae and the recognition sequences are the FRT sequences which flank all or part of an inserted gene or its regulatory elements.

13. A method as claimed in claim 4, wherein the disrupter gene is a recombinase or a related enzyme of similar function adapted to excise a nucleotide sequence flanked by recombinase recognition sequences, and wherein the recombinase gene is the Cre gene of bacteriophage P1 and its recognition sequences are the lox sequences which flank all or part of an inserted gene or its regulatory elements.

14. A method as claimed in claim 4, wherein the disrupter gene is a recombinase or a related enzyme of similar function adapted to excise a nucleotide sequence flanked by recombinase recognition sequences, and wherein the recombinase gene is the Activator transposase of Zea mays.

15. A method according to any one of claims 1 to 14 wherein the inducible promoter is the promoter of the gene encoding the 27 kd protein of glutathione-S-transferase II.

16. A method as claimed in any one of claims 1 to 14 wherein the said inducible promoter comprises the promoter of the AlcA gene, the system further comprising a gene capable of expressing the AlcR protein, alcA and alcR being obtainable from Aspergillus.

17. A method according to any one of claims 1 to 16 wherein the expression system comprises a repressor protein gene and wherein the repressor protein gene encodes the lac repressor or a repressor used by 434, P22 or lambdabacteriophages.

18. A method as claimed in any one of claims 1 to 16 which comprises a repressor protein gene and wherein the repressor protein is the tet repressor.

19. A method as claimed in claim 1, wherein the disrupter gene of the expression system encodes barnase and the gene encoding the inhibitor of the disrupter gene contains the coding region of the barstar gene which on expression produces a protein inhibitor of barnase.

20. A method for preventing pre-harvest sprouting of a plant, said method comprising transforming a plant with an expression system functional in a plant and comprising:
(a) an inducible promoter sequence responsive to the presence or absence of an exogenous chemical inducer;
(b)
either (i) a gene encoding a repressor protein under control of the said inducible promoter,
or (ii) a gene encoding an inhibitor of the disrupter gene specified at (d) below under control of the said inducible promoter;
(c) a plant developmental gene promoter sequence activated at the germination or early seed development stage of a plant, which, in the case of (b)(i) above, includes an operator sequence recognised by the said repressor protein, the presence of which inactivates the said plant developmental gene promoter;
(d) a gene encoding a protein-disrupter of plant germination, under the control of the said plant developmental gene promoter sequence;
whereby the presence or absence of the exogenous chemical inducer controls whether a seed of the said plant will germinate.

21. A method as claimed in claim 20 wherein the said inducible promoter sequence is functionally linked to and controls a repressor protein gene and in which the disrupter gene promoter includes an operator sequence recognised by the said repressor protein, so that in the presence of the inducer the repressor protein is produced which interacts with the operator sequence disabling the plant developmental gene promoter and inhibiting expression of the disrupter gene.

22. A method according to claim 20 or claim 21 wherein the disrupter gene encodes a nucleotide sequence adapted to inhibit an endogenous plant gene which is essential to germination.

23. A method according to any one of claims 20 to 22 wherein the presence of endogenous chemical will allow the seed to germinate.

## Patentansprüche

1. Verfahren zur Eindämmung des genetischen Materials einer Pflanze, wobei das Verfahren das Transformieren einer Pflanze mit einem Expressionssystem umfaßt, das in einer Pflanze funktionsfähig ist und folgendes umfaßt:
(a) eine induzierbare Promotorsequenz, die auf die Gegenwart oder Abwesenheit eines exogenen chemischen Induktors anspricht;
(b)
entweder (i) ein Gen, das ein Repressorprotein codiert, unter der Kontrolle des induzierbaren Promotors,
oder (ii) ein Gen, das einen Inhibitor des nachfolgend bei (d) genannten Disruptor-Gens codiert, unter der Kontrolle des induzierbaren Promotors;
(c) eine pflanzliche Entwicklungsgen-Promotorsequenz, die in einer ausgewählten Stufe der pflanzlichen Entwicklung aktiviert wird, die im Falle von (b)(i) oben eine Operatorsequenz einschließt, die durch das Repressorprotein erkannt wird, dessen Gegenwart den pflanzlichen Entwicklungsgen-Promotor inaktiviert;
(d) ein Gen, das einen Proteindisruptor einer Pflanzeneigenschaft codiert, wobei die Eigenschaft entweder wesentlich für das pflanzliche Wachstum oder die Entwicklung ist, oder die eine Eigenschaft ist, die aus der Gen-Insertion resultiert, unter der Kontrolle der pflanzlichen Entwicklungsgen-Promotorsequenz;
wobei die Gegenwart oder Abwesenheit des exogenen chemischen Induktors steuert, ob die Eigenschaft in der Pflanze gezeigt wird.

2. Verfahren gemäß Anspruch 1, worin die durch das Expressionssystem gesteuerte Pflanzeneigenschaft wesentlich für das pflanzliche Wachstum ist, wobei die Gegenwart oder Abwesenheit des exogenen chemischen Induktors entweder das Wachstum bis zur Reife erlaubt oder die Verlangsamung oder das Anhalten des Wachstums in der ausgewählten Stufe verursacht.

3. Verfahren gemäß Anspruch 1 oder 2, worin die induzierbare Promotorsequenz funktionsfähig mit einem Repressorprotein-Gen verbunden ist und dieses steuert, und worin der Disruptorgen-Promotor eine durch das Repressorprotein erkannte Operatorsequenz einschließt, so daß in Gegenwart des Induktors das Repressorprotein erzeugt wird, das mit der Operatorsequenz wechselwirkt, wodurch der pflanzliche Entwicklungsgen-Promotor inaktiviert und die Expression des Disruptor-Gens gehemmt wird.

4. Verfahren gemäß einem der Ansprüche 1 bis 3, worin das Disruptor-Gen entweder (i) ein Zellgift, das die Zellfunktion stört, was zum Zelltod führt, oder (ii) eine Rekombinase oder ein verwandtes Enzym ähnlicher Funktion, das dazu angepaßt ist, eine Nukleotidsequenz auszuschneiden, die von Rekombinase-Erkennungssequenzen flankiert ist, oder (iii) eine Nukleotidsequenz codiert, die dazu angepaßt ist, ein endogenes Pflanzen-Gen, das wesentlich für die pflanzliche Entwicklung ist, oder ein insertiertes Gen zu hemmen, das eine gewünschte Eigenschaft auf die Pflanze überträgt.

5. Verfahren gemäß Anspruch 4, worin das Disruptor-Gen eine Nukleotidsequenz codiert, die dazu angepaßt ist, ein endogenes Pflanzen-Gen, das wesentlich für die pflanzliche Entwicklung ist, oder ein insertiertes Gen zu hemmen, das eine gewünschte Eigenschaft auf die Pflanze überträgt, und worin die Nukleotidsequenz in antisense-Orientierung zum zu hemmenden Gen ist und weniger als der vollen Länge des zu hemmenden Gens entspricht.

6. Verfahren gemäß Anspruch 5, worin das zu hemmende Gen ein endogenes Pflanzen-Gen ist, das wesentlich für die Saatkeimung oder frühe Keimlingsentwicklung ist.

7. Verfahren gemäß Anspruch 6, worin das zu hemmende oder auszuschneidende Gen ein α-Amylase-Gen ist.

8. Verfahren gemäß einem der Ansprüche 1 bis 7, worin die pflanzliche Entwicklungspromotor-Sequenz der Promotor eines Gens ist, das normalerweise während der Keimung oder frühen Keimlingsentwicklung aktiv ist.

9. Verfahren gemäß Anspruch 8, worin der Promotor der Promotor des Malatsynthase-Gens ist.

10. Verfahren gemäß Anspruch 8, worin der Promotor der Promotor des Germin-Gens ist.

11. Verfahren gemäß Anspruch 8, worin der Promotor aus der Gruppe ausgewählt ist, die aus den Gen-Promotoren von glyoxysomalen Enzym-Genen, Aleuronschicht-Genen und Carboxypeptidase-Genen besteht.

12. Verfahren gemäß Anspruch 4, worin das Rekombinase-Gen das FLP-Gen des 2-Mikron-Plasmids von Saccharomyces cerevisiae ist und die Erkennungssequenzen die FRT-Sequenzen sind, die das gesamte oder einen Teil eines insertierten Gens oder seiner regulatorischen Elemente flankieren.

13. Verfahren gemäß Anspruch 4, worin das Disruptor-Gen eine Rekombinase oder ein verwandtes Enzym ähnlicher Funktion ist, das dazu angepaßt ist, eine Nukleotidsequenz auszuschneiden, die von Rekombinase-Erkennungssequenzen flankiert ist, und worin das Rekombinase-Gen das Cre-Gen des Bacteriophagen P1 ist und seine Erkennungssequenzen die lox-Sequenzen sind, die das gesamte oder einen Teil eines insertierten Gens oder seiner regulatorischen Elemente flankieren.

14. Verfahren gemäß Anspruch 4, worin das Disruptor-Gen eine Rekombinase oder ein verwandtes Enzym ähnlicher Funktion ist, das dazu angepaßt ist, eine Nukleotidsequenz auszuschneiden, die von Rekombinase-Erkennungssequenzen flankiert ist, und worin das Rekombinase-Gen die Activator-Transposase von Zea mays ist.

15. Verfahren gemäß einem der Ansprüche 1 bis 14, worin der induzierbare Promotor der Promotor des Gens ist, das das 27 kd-Protein von Glutathion-S-transferase II codiert.

16. Verfahren gemäß einem der Ansprüche 1 bis 14, worin der induzierbare Promotor den Promotor des AlcA-Gens umfaßt, wobei das System ferner ein zur Expression des AlcR-Proteins fähiges Gen umfaßt, und wobei alcA und alcR aus Aspergillus erhältlich sind.

17. Verfahren gemäß einem der Ansprüche 1 bis 16, worin das Expressionssystem ein Repressorprotein-Gen umfaßt und worin das Repressorprotein-Gen den lac-Repressor oder einen von 434-, P22- oder lamda-Bacteriophagen verwendeten Repressor codiert.

18. Verfahren gemäß einem der Ansprüche 1 bis 17, welches ein Repressorprotein-Gen umfaßt, und worin das Repressorprotein der tet-Repressor ist.

19. Verfahren gemäß Anspruch 1, worin das Disruptor-Gen des Expressionssystems Barnase codiert und das Gen, das den Inhibitor des Disruptor-Gens codiert, die codierende Region des Barstar-Gens enthält, das bei Expression einen Proteininhibitor von Barnase erzeugt.

20. Verfahren zur Verhinderung des Vorernte-Aufgehens einer Pflanze, wobei das Verfahren das Transformieren einer Pflanze mit einem Expressionssystem umfaßt, das in einer Pflanze funktionsfähig ist und umfaßt:
(a) eine induzierbare Promotorsequenz, die auf die Gegenwart oder Abwesenheit eines exogenen chemischen Induktors anspricht;
(b) entweder
(i) ein Gen, das ein Repressorprotein codiert, unter der Kontrolle des induzierbaren Promotors,
oder (ii) ein Gen, das einen Inhibitor des bei (d) unten angegebenen Disruptor-Gens codiert, unter der Kontrolle des induzierbaren Promotors;
(c) eine pflanzliche Entwicklungsgen-Promotorsequenz, die bei der Keimungs- oder frühen Saatentwicklungsstufe einer Pflanze aktiviert wird, die im Falle (b)(i) oben eine Operatorsequenz einschließt, die durch das Repressorprotein erkannt wird, dessen Gegenwart den pflanzlichen Entwicklungsgen-Promotor inaktiviert;
(d) ein Gen, das einen Proteindisruptor der pflanzlichen Keimung codiert, unter der Kontrolle der pflanzlichen Entwicklungsgen-Promotorsequenz;
wobei die Gegenwart oder Abwesenheit des exogenen chemischen Induktors steuert, ob ein Samen der Pflanze keimen wird.

21. Verfahren gemäß Anspruch 20, worin die induzierbare Promotorsequenz funktionsfähig mit einem Repressorprotein-Gen verbunden ist und dieses steuert, und worin der Disruptorgen-Promotor eine Operatorsequenz einschließt, die durch das Repressorprotein erkannt wird, so daß in Gegenwart des Induktors das Repressorprotein erzeugt wird, welches mit der Operatorsequenz wechselwirkt, wodurch der pflanzliche Entwicklungsgen-Promotor inaktiviert und die Expression des Disruptor-Gens gehemmt wird.

22. Verfahren gemäß Anspruch 20 oder 21, worin das Disruptor-Gen eine Nukleotidsequenz codiert, die dazu angepaßt ist, ein endogenes Pflanzen-Gen zu hemmen, das wesentlich für die Keimung ist.

23. Verfahren gemäß einem der Ansprüche 20 bis 22, worin die Gegenwart von endogener Chemikalie das Keimen des Samens erlauben wird.

## Revendications

1. Procédé pour contenir un matériau génétique d'une plante, ledit procédé comprenant la transformation d'une plante avec un système d'expression fonctionnel dans une plante, et comprenant :
(a) une séquence d'un promoteur inductible sensible à la présence ou à l'absence d'un inducteur chimique exogène ;
(b)
soit (i) un gêne codant une protéine répressive sous le contrôle dudit promoteur inductible ;
soit (ii) un gêne codant un inhibiteur du gêne disrupteur spécifié au (d) ci-dessous sous le contrôle dudit promoteur inductible ;
(c) une séquence de promoteur de gêne de développement d'une plante activée à un stade sélectionné du développement de la plante, qui, dans le cas de (b) (i) ci-dessus, comprend une séquence opératrice reconnue par ladite protéine répressive, dont la présence désactive ledit promoteur de gêne de développement de la plante ;
(d) un gêne codant une protéine disruptive d'une caractéristique de plante, laquelle caractéristique est soit essentielle à la croissance ou au développement de la plante, ou qui est une caractéristique résultant de l'insertion de gêne, sous le contrôle de ladite séquence de promoteur de gêne de développement de la plante ;
moyennant quoi la présence ou l'absence de l'inducteur chimique exogène contrôle si ladite caractéristique est affichée dans la plante.

2. Procédé selon la revendication 1 dans lequel la caractéristique de la plante contrôlée par le système d'expression est essentiel à la croissance de la plante, moyennant quoi la présence ou l'absence de l'inducteur chimique exogène permet soit la croissance jusqu'à la maturité ou provoque l'arrêt de la croissance ou l'arrête audit stade sélectionné.

3. Procédé selon la revendication 1 ou 2 dans lequel ladite séquence de promoteur inductible est fonctionnellement lié à et contrôle un gêne de protéine répressive et dans lequel le promoteur de gêne disrupteur comprend une séquence opératrice reconnue par ladite protéine répressive, de sorte qu'en présence de l'inducteur, la protéine répressive est produite et interagit avec la séquence opératrice en interrompant le promoteur de gêne de développement de la plante et en inhibant l'expression du gêne disrupteur.

4. Procédé selon l'une quelconque des revendications 1 à 3, dans lequel le gène disrupteur code soit (i) une cytotoxine qui arrête la fonction cellulaire, entraînant la mort de la cellule, soit (ii) une recombinase ou un enzyme relatif de la fonction similaire adaptée pour exciser une séquence de nucléotides flanquée de séquences de reconnaissance de la recombinase, soit (iii) une séquence de nucléotides adaptée pour inhiber un gêne de plante endogène qui est essentiel au développement de la plante, soit un gêne rapporté conférant la caractéristique désirée à la plante.

5. Procédé selon la revendication 4, dans lequel le gène disrupteur code une séquence de nucléotides adaptée pour inhiber un gène de plante endogène qui est essentiel au développement de la plante ou un gène conférant une caractéristique désirée à la plante, et dans lequel ladite séquence de nucléotides est dans une orientation antisens par rapport au gêne à inhiber, et correspond à moins de la longueur totale dudit gêne à inhiber.

6. Procédé selon la revendication 5, dans lequel le gêne à inhiber est un gêne de plante endogène essentiel pour faire germer les graines ou le développement de la germination précoce.

7. Procédé selon la revendication 6, dans lequel le gène à inhiber ou à exciser est un gène de l'α-amylase.

8. Procédé selon l'une quelconque des revendications 1 à 7, dans lequel ladite séquence de promoteur de développement de la plante est un promoteur de gêne normalement actif pendant la germination ou le développement de la germination précoce.

9. Procédé selon la revendication 8, dans lequel ledit promoteur est le promoteur de gène de la malate synthase.

10. Procédé selon la revendication 8, dans lequel ledit promoteur est le promoteur du gène de la germine.

11. Procédé selon la revendication 8, dans lequel ledit promoteur est choisi parmi le groupe constitué par les promoteurs de gène des gènes de l'enzyme de glyoxysome, des gènes de la couche d'aleurone et des gènes de la carboxypeptidase.

12. Procédé selon la revendication 4, dans lequel le gène de la recombinase est le gène FLP du plasmide de 2 microns de Saccharomyces cerevisiae et les séquences de reconnaissance sont les séquences de FRT qui flanquent la totalité ou une partie d'un gène inséré ou ses éléments régulateurs.

13. Procédé selon la revendication 4, dans lequel le gène disrupteur est une recombinase ou un enzyme relatif de la fonction similaire adaptée pour exciser une séquence de nucléotides par des séquences de reconnaissance de recombinase, et dans lequel le gêne est le gène Cre du bactériophage P1 et ses séquences de reconnaissance sont les séquences lox qui flanquent la totalité ou une partie d'un gène inséré ou ses éléments régulateurs.

14. Procédé selon la revendication 4, dans lequel le gène disrupteur est une recombinase ou un enzyme relatif d'une fonction similaire adaptée pour exciser une séquence de nucléotides flanquée par des séquences de reconnaissance de recombinase, et dans lequel le gêne de la recombinase est l'activateur de transposase de Zea mays.

15. Procédé selon l'une quelconque des revendications 1 à 14, dans lequel le promoteur inductible est le promoteur du gène codant la protéine de la glutathione-S-transférase II de 27 kd.

16. Procédé selon l'une quelconque des revendications 1 à 14, dans lequel ledit promoteur inductible comprend le promoteur du gène AlcA, le système comprenant en outre un gène capable d'exprimer la protéine AlcR, alcA et alcR pouvant être obtenu à partir d'Aspergillus.

17. Procédé selon l'une quelconque des revendications 1 à 16, dans lequel le système d'expression comprend un gène de la protéine répressive et dans lequel le gêne de la protéine répressive code le répresseur lac ou un répresseur utilisé par les bactériophages 434, P22 ou lambda.

18. Procédé selon l'une quelconque des revendications 1 à 16, qui comprend un gêne de protéine répressive et dans lequel la protéine répressive est le répresseur tet.

19. Procédé selon la revendication 1, dans lequel le gène disrupteur du système d'expression code la barnase, et le gêne codant l'inhibiteur du gêne disrupteur contient la région codante du gène barstar qui produit, à l'expression, un inhibiteur de protéine de la barnase.

20. Procédé pour empêcher l'émergence d'une plante avant la récolte, ledit procédé comprenant la transformation d'une plante avec un système fonctionnel d'expression dans une plante et comprenant :
(a) une séquence d'un promoteur inductible sensible à la présence ou à l'absence d'un inducteur chimique exogène ;
(b)
soit (i) un gêne codant une protéine répressive sous le contrôle dudit promoteur inductible ;
soit (ii) un gêne codant un inhibiteur du gêne disrupteur spécifié au (d) ci-dessous sous le contrôle dudit promoteur inductible ;
(c) une séquence de promoteur de gêne de développement d'une plante activée à la germination ou au stade du développement de la germination précoce de la plante, qui, dans le cas de (b) (i) ci-dessus, comprend une séquence opératrice reconnue par ladite protéine répressive, la présence de laquelle désactive ledit promoteur de gêne de développement de la plante ;
(d) un gêne codant une protéine disruptive de la germination de la plante sous le contrôle de ladite séquence de promoteur de gêne de développement de la plante;
moyennant quoi la présence ou l'absence de l'inducteur chimique exogène contrôle si la germination de ladite plante a lieu.

21. Procédé selon la revendication 20 dans lequel ladite séquence de promoteur inductible est fonctionnellement lié à et contrôle un gêne de protéine répressive et dans lequel le promoteur de gêne disrupteur comprend une séquence opératrice reconnue par ladite protéine répressive, de sorte qu'en présence de l'inducteur, la protéine répressive est produite et interagit avec la séquence opératrice en interrompant le promoteur de gêne de développement de la plante et en inhibant l'expression du gêne disrupteur.

22. Procédé selon la revendication 20 ou 21 dans lequel le gêne disrupteur code une séquence de nucléotides adaptée pour inhiber un gêne de plante qui est essentiel à la germination.

23. Procédé selon l'une quelconque des revendications 20 à 22 dans lequel la présence de produit chimique endogène permet la germination de la graine.
